# EUROPEAN PATENT APPLICATION

(11) **EP 4 497 369 A1**
(43) Date of publication of application: **29.01.2025**
(21) Application number: 24158205.5
(22) Date of filing: 16.02.2024
(51) Int. Cl.: A61B 1/00, A61B 1/267

(54) **METHOD AND SYSTEM FOR MEDICAL ENDOSCOPIC IMAGING ANALYSIS AND MANIPULATION**

(30) Priority: 25.07.2023 US 202363528711 P
(71) Applicant: Olympus Medical Systems Corporation, Hachioji-shi Tokyo 192-8507 (JP)
(72) Inventor: LAZZARI, Matteo, 22303 Hamburg (IT); ARIYOSHI, Daiki, Chofu City (JP); SAKAI, Yuji, Kodaira-city, 187-0011 (JP)
(74) Representative: Seemann & Partner Patentanwälte mbB

(57) **Abstract**

An endoscopic medical imaging analysis and manipulation method comprising: Capturing endoscopic images of laryngeal or pharyngeal tissue structures during examination of the larynx using an endoscope inserted through a patient's nose or mouth, Feeding the captured endoscopic images to two instances of artificial intelligences, a first of which having been trained to identify suspicious areas of laryngeal or pharyngeal tissue structures showing signs of alterations from healthy laryngeal or pharyngeal tissue and a second of which having been trained to detect and classify abnormalities in the mucosal layer or the tissue of the larynx or pharynx, Overlaying the captured endoscopic images with one or more markings indicating an area or areas indicated by the first instance of artificial intelligence as suspicious, and Displaying the overlaid captured endoscopic images on a monitor, together with information provided by the second instance of artificial intelligence.

## Description

The present disclosure relates to a method and a system for medical endoscopic imaging analysis and manipulation.

Laryngeal biopsies are a routine diagnostic procedure in ENT (ear, nose, throat) medicine to evaluate and examine the larynx and other parts of the procedure is performed using a flexible or rigid endoscope inserted through the patient's nose or mouth. This allows the visualization of the larynx, pharynx and surrounding tissue and identification of any suspicious areas or changes in the mucosal layer that may indicate inflammation or cancer.

In busy clinics, this procedure is often done in an office setting without anesthesia and takes only a few minutes. However, this time constraint and lack of patient cooperation can make it challenging for doctors to fully inspect the anatomy and focus on small details that may indicate early disease. Additionally, the anatomy of elderly patients can be more difficult to examine, making it challenging to explore all the mucosa and identify potential signs of disease.

It is common for certain types of lesions to go unnoticed in medical examinations, especially when specialists are not readily available. This is often the case in hospitals lacking such specialists, since specialists are mainly located in larger university centers. In the field of ENT, early detection is particularly important in order to preserve the functionality of the larynx and improve patient outcome. However, traditional methods and human observation can open miss these early-stage lesions, often due to lack of experience.

The main problem with early diagnosis of cancerous or benign lesions in ENT is to accurately and efficiently identify and detect potential lesions or abnormal tissue in the early stages of the disease. This is particularly challenging because early-stage ENT cancers may not be visible using traditional methods such as white light endoscopy and may be missed by human observation alone. The use of Narrow Band Imaging (NBI) can aid in the identification of lesions. NBI is a medical imaging technique that utilizes specific wavelengths of light to enhance the visualization of blood vessels, mucosa and other structures. This technology is widely used in the field of ENT to improve the visualization of vessels and its efficiency is scientifically proven. The use of such imaging enhancement can aid in the early detection of lesions or abnormal tissue that may indicate inflammation or cancer. However, the effectiveness of this technology is contingent upon the skill level of the user interpreting the images.

Additionally, the lack of expertise and experience among medical professionals in identifying early-stage ENT cancers can occur, which can lead to delayed diagnoses or misdiagnoses. Furthermore, the complexity of the anatomy of the ENT region and the high variability of the lesions make it challenging to develop a standard diagnostic procedure. This can lead to a lack of consistency in diagnostic results across different hospitals and clinics.

The invention being described here aims to address these challenges by supporting doctors and improving early detection and diagnosis. Therefore, it is an object to provide a method and a system that aid medical practitioners in the field of ENT in providing better diagnoses and patient outcomes.

Such object can be solved by an endoscopic medical imaging analysis and manipulation method, the method comprising:
capturing endoscopic images of laryngeal, pharyngeal and/or surrounding tissue structures during examination of the larynx and/or pharynx using an endoscope inserted through a patient's nose or mouth,
feeding the captured endoscopic images to two instances of artificial intelligences, a first of which having been trained to identify suspicious areas of laryngeal, pharyngeal and/or surrounding tissue structures showing signs of alterations from healthy laryngeal, pharyngeal and/or surrounding tissue and a second of which having been trained to detect and classify abnormalities in the mucosal layer or the tissue of the larynx,
overlaying the captured endoscopic images with one or more markings indicating an area or areas indicated by the first instance of artificial intelligence as suspicious, and
displaying the overlaid captured endoscopic images on a monitor, together with information provided by the second instance of artificial intelligence.

The dual-A-assisted diagnostic tool addresses the above-mentioned challenges and improve the accuracy and efficiency of early diagnosis in ENT, such as cancerous abnormalities, enabling ENT doctors to better perform triage and treatment during endoscopies of the upper aerodigestive tract, which in turn leads to better outcomes for patients.

The two-stage AI assisted method enhances the assistance a system can render to a medical professional carrying out an examination by providing him with a first-stage general overview suitable for finding any suspicious areas and a second-stage detailed analysis of such suspicious areas, so that the professional can switch between assisted overview and assisted detailed analysis as the examination requires.

In embodiments, the endoscopic images are one of WLI images, NBI images and a mix of WLI images and NBI images. For example, the first stage might be done with WLI (white light imaging) images, whereas the detailed second stage analysis may be performed using NBI (narrow band imaging). Narrow band imaging (NBI) is a medical imaging technique that utilizes specific wavelengths of light to enhance the visualization of blood vessels, mucosa and other structures. This technology is widely used in the field of ENT (Ear, Nose, and Throat) to improve the visualization of vessels. NBI images that have been shown to be superior to white light imaging (WLI) in highlighting abnormal mucosal or tissue structures in the larynx and surrounding tissues.

Numerous clinical studies have shown a correlation between different vascular patterns and various disease states. Alterations in blood vessel morphology and density have been shown to reflect the severity of the disease. At this moment, four different vascular classifications that are based on NBI have been widely adopted in the field of otolaryngology. These classifications aid in the visual identification of the type of lesion and disease. The presently disclosed method and system are directed to enhancing the diagnostic capabilities of otolaryngology physicians by incorporating an instance of artificial intelligence (Al) and machine learning (ML) techniques in conjunction with NBI, to guide targeted biopsy procedures and thereby improve patient outcomes.

The overlays of the inventive method, to be displayed for example within a specific GUI (Graphical user interface), will identify lesions and other abnormalities with higher accuracy rate and earlier than human observers could, since small vessels that might be part of a lesion are often not visible to the human eye. If missed, these can lead to recurrences. Together with the fact that NBI images are used, physicians in otolaryngology are aided by the overlay, even if they are less experienced, e.g., for a lack of cases in less populated areas.

The system used in the method of the present disclosure has two different modules with two different instances of artificial intelligence, the first one with the go to support the doctor to detect abnormal tissue and the second module to detect and characterize lesions. The second module may be trained to perform the detection of lesions by itself, or it may be fed with the findings of the first module and proceed with the characterization of lesions detected and individualized by the first module.

In embodiments, the first instance of an artificial intelligence may be a convolutional neural network (CNN) having a classifier, the CNN having been trained by at least one of supervised and unsupervised learning of a multitude of endoscopic images of laryngeal, pharyngeal and/or surrounding tissue structures to classify suspicious areas of laryngeal, pharyngeal and/or surrounding tissue in the captured endoscopic images.

The term "unsupervised learning" typically describes a process wherein the neural network is trained using only images of healthy laryngeal tissue. Any region that does not conform to what the neural network has learned as being healthy will be marked suspicious. Supervised training takes the training a step further. Diseased areas are marked and classified beforehand in the training data so that the neural network is trained to not only identify suspicious areas, but also provide an estimate about what kind of lesion is shown in an NBI image.

In further embodiments, the second instance of an artificial intelligence may be a convolutional neural network (CNN) having a classifier, the CNN having been trained by supervised learning of a multitude of pre-classified endoscopic images of laryngeal, pharyngeal and/or surrounding tissue structures to classify suspicious areas of laryngeal tissue in the captured endoscopic images according to type and/or severity of abnormalities in the suspicious areas. In this case, the second instance of an artificial intelligence may have been trained to identify alterations from healthy laryngeal, pharyngeal and/or surrounding tissue structures as suspicious stemming from one or more of cancerous and benign lesions, blood vessel morphologies, blood vessel densities, vascular patterns, and structure of the mucosal surface. By this, the disclosed algorithm utilizes image analysis techniques to aid otolaryngology physicians in the identification of vascular patterns, density, morphology, and structure on the mucosal surface. By providing such information, the algorithm supports the diagnostic capabilities of the physician and assists in the identification of potential pathology.

The overlays are created as rectangles or outlines in embodiments. The overlays are the result of the analysis carried out by the first instance of artificial intelligence and support the detection of abnormal mucosal layers with their visual identification. A doctor may indi-viduate a suspicious area faster if it is visualized after having been recognized by, for example, changes in the vascular morphology. In case of multiple suspicious areas, there may be several markings, such as rectangles, to avoid missing a spot. The markings thereby serve to enhance and support the surveillance of the entire anatomy, thereby reducing the risk of missing any potential abnormalities that could lead to the development of cancer. The number of rectangles may vary depending on the number of suspicious areas found. The algorithm may add new overlaid rectangles depending on mucosal changes that are discovered during an endoscopy.

The rectangles or outlines may, in embodiments, be adjustable based on the movement of the user, thereby providing a more dynamic and personalized experience. The adjustment with respect to movement may be implemented, for example, by movement analysis in the imagery, where shifts or movements in three dimensions are identified from comparisons between subsequent frames of the endoscopic imaging, or where the identification of such shifts may be aided by motion sensors inside the endoscope. Additionally, the user may have the ability to customize the overlay display, such as highlighting the target lesion with a continuous line and surrounding lesions with a dotted line. This feature aids the doctor in focusing on the target lesion and getting a closer look to identify more details, thereby reducing the risk of overlooking small lesions that could lead to recurrences.

A live measure of classification accuracy by the first instance of artificial intelligence and or the second instance of artificial intelligence may be displayed, the measure of accuracy in particular based on factors such as the distance of the endoscope from the lesion, the angulation of the lengths, and clarity of the image. The measure of accuracy may be displayed as numbers, such as percentages, as a color coding, for example. The artificial intelligence algorithms may be based on various factors, including distance, image quality, light reflection, and an available data set. Such parameters may be used to determine the real-time accuracy of the instance of artificial intelligence.

The first instance of artificial intelligence or the second instance of artificial intelligence may in embodiments be trained to identify image defects that necessitate an adjustment or cleaning of the endoscope lens, and a user is notified of a suggestion to adjust or clean the endoscope lens in the case of the occurrence of such image defects, if the image is not sufficiently clear.

The artificial intelligence functionality may be activated and deactivated during an endoscopy procedure according to need. Any one or both of the instances of artificial intelligence may be designed to continuously learn from you images, which may be classified while being produced by the physicians performing the examination in that the physicians confirm, alter or add findings with respect to suspicious areas or classifications.

The user may be able to program the rectangles or other markings with different colors, for example green, yellow and red, based on the severity of a lesion using a recent WHO nomenclature. For example, the classification may depend on preset databased pictures and be based on recognized guidelines that are already in use.

A graphic user interface (GUI) may be used in which patient information and other information, such as settings or setting preferences, and lesion specific information, may be displayed side-by-side with the endoscopic images. The GUI may also indicate if a CAD mode is active, or WLI or NBI conditions. The complete interface may also be recorded.

The second instance of artificial intelligence may be trained to utilize vascular classifications in NBI images in order to gain more accurate information in embodiments. The second instance of artificial intelligence may also be trained to estimate the size of a lesion and to provide an estimation of its length. The size information may then be reconstructed into a two-dimensional lesion map providing a clearer representation of the anatomical area in question. This will aid the doctors in better identifying and mapping suspicious areas.

The object underlying the present disclosure is also achieved by an endoscopic medical imaging analysis und manipulation system comprising a video endoscope suited to be fed through a patient's mouth or nose for laryngeal and/or pharyngeal examination, an image analyzer connected to the video endoscope for receiving endoscopic images from the endoscope, the image analyzer having a first instance of an artificial intelligence trained to identify suspicious areas of laryngeal, pharyngeal and/or surrounding tissue structures showing signs of alterations from healthy laryngeal, pharyngeal and/or surrouding tissue and a second instance of artificial intelligence having been trained to detect and classify abnormalities in the mucosal layer or the tissue of the larynx, pharynx or surrounding tissue, the image analyzer further being configured to overlay, the captured endoscopic images with a marking indicating areas indicated by the instance of artificial intelligence as suspicious, and a monitor connected to the image analyzer for displaying endoscopic images provided by the image analyzer, together with information provided by the second instance of artificial intelligence.

The system embodies the same features and advantages as the above-described endoscopic medical imaging analysis and manipulation method of the present disclosure. It may include a light source capable of producing light for WLI as well as for NBI.

In embodiments, the first instance of an artificial intelligence is a convolutional neural network (CNN) having a classifier, the CNN having been trained by at least one of supervised and unsupervised learning of a multitude of endoscopic images of laryngeal, pharyngeal and/or surrounding tissue structures to classify suspicious areas of laryngeal, pharyngeal and/or surrounding tissue in the captured endoscopic images, and/or the second instance of an artificial intelligence is a convolutional neural network (CNN) having a classifier, the CNN having been trained by supervised learning of a multitude of pre-classified endoscopic images of laryngeal, pharyngeal and/or surrounding tissue structures to classify suspicious areas of laryngeal, pharyngeal and/or surrounding tissue in the captured endoscopic images according to type and/or severity of abnormalities in the suspicious areas.

In a further embodiment, the type of abnormalities include one or more of cancerous and benign lesions, cancerous and benign blood vessel morphologies, cancerous and benign blood vessel densities, cancerous and benign vascular patterns, and cancerous and benign structure of the mucosal surface.

The overlays may be adjustable based on the movements of the user in embodiments.

In further embodiments, a live measure of classification accuracy by the first instance of artificial intelligence and/or the second instance of artificial intelligence is displayed, in particular based on factors such as the distance of the endoscope from the lesion, the angulation of the lengths, and clarity of the image.

The first instance of artificial intelligence or the second instance of artificial intelligence may be trained to identify image defects that necessitate an adjustment or cleaning of the endoscope lens or cleaning of the anatomical area, and a user may be notified of a suggestion to adjust or clean the endoscope lens or to clean the anatomical area in the case of the occurrence of such image defects.

In embodiments, any one or both of the instances of artificial intelligence are designed to continuously learn from new images. The new images may be classified while being produced by a physician performing the examination through confirming, altering or adding findings with respect to suspicious areas or classifications.

In an embodiment, the second instance of artificial intelligence is trained to estimate the size of a lesion and to provide an estimation of its length, the size information in particular being used to produce a two-dimensional lesion map of the anatomical area.

The object underlying the present disclosure is also achieved by a computer program stored on a non-volatile medium, the computer program being designed to perform any of the embodiments of the method of the present disclosure when executed on an image processor of the system of the present disclosure.

Further features will become evident from the description of embodiments, together with the claims and the appended drawings. Embodiments can fulfill individual features or a combination of several features.

The embodiments described below, without restricting the general intent of the invention, based on exemplary embodiments, wherein reference is made expressly to the drawings with regard to the disclosure of all details that are not explained in greater detail in the text.

In the drawings:
- Fig. 1: illustrates a schematic representation of the steps of the image analysis and manipulation method,
- Fig. 2: illustrates a schematic representation of an embodiment of an instance of an artificial intelligence,
- Fig. 3: illustrates an embodiment of a GUI representing the result of the present method,
- Fig. 4: illustrates another embodiment of a GUI representing the result of the present method,
- Fig. 5: illustrates representations of training data for the instances of artificial intelligence and
- Fig. 6: illustrates a schematic representation of a system according to the present disclosure.

In the drawings, the same or similar types of elements or respectively corresponding parts are provided with the same reference numbers in order to prevent the item from needing to be reintroduced.

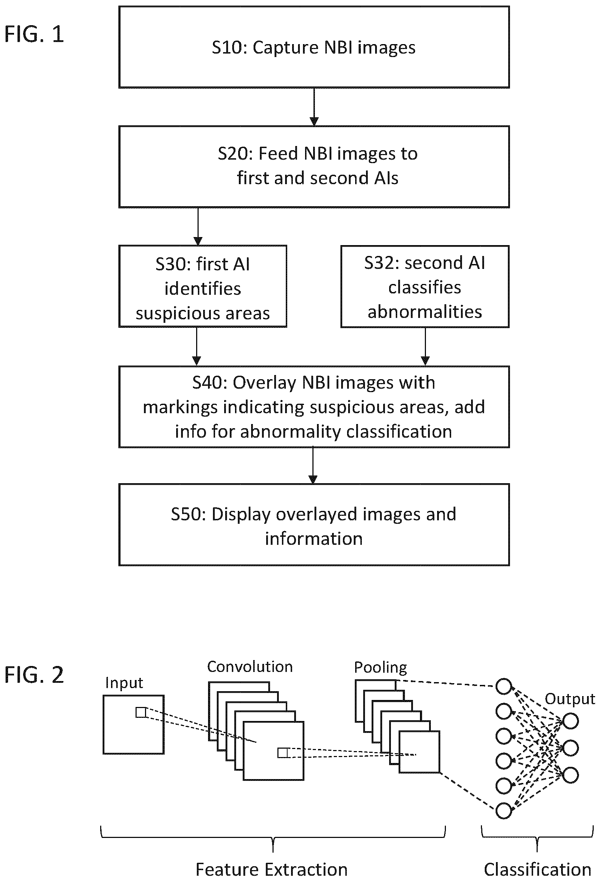

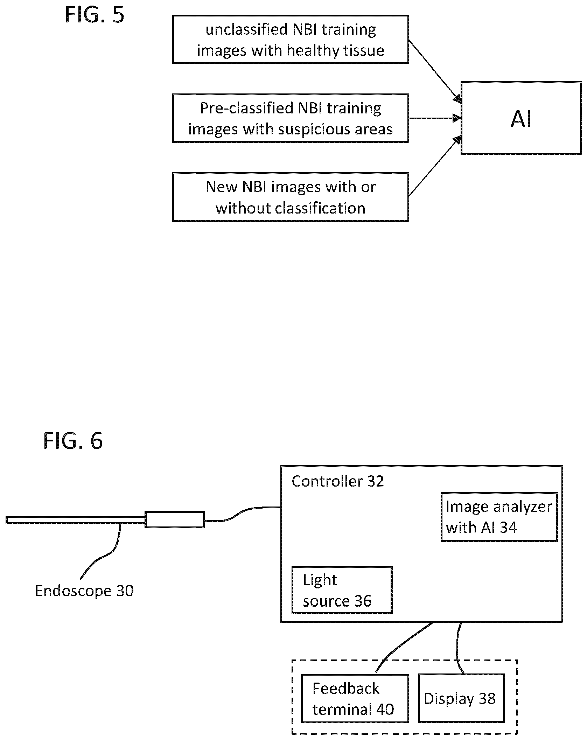

Fig. 1 displays a schematic representation of the steps of the image analysis and manipulation method, which can be carried out using a system, an exemplary embodiment of which is shown Fig. 5. In step S10, images from narrow band imaging (NBI) or from white light imaging of the larynx of the patient are captured using a video endoscope, which may have an image sensor at its distal end, or maybe of a different type with an image sensor at the proximal end or a separate camera head attached to a purely optical endoscope. These are sent to an image processor in step S20 that runs two instances of artificial intelligence that have been trained with WLI and/or NBI images of the larynx to identify suspicious areas in the mucous surface or the tissue of the larynx and to classify abnormalities according to type and severity, respectively. In steps S30 and S32, the first and second instance of artificial intelligence, respectively, process the images and identifies suspicious areas according to their respective training. The identification of suspicious areas can be done by classifying such areas as suspicious.

The first instance of artificial intelligence provides the suspicious areas as output, so that in step S40, the image analyzer is able to overlay the images with markings indicating the suspicious areas found by the first instance of artificial intelligence. The type and severity information, as well as any additional related information provided be the second instance of artificial intelligence are added to the display. The overlaid images are then displayed to personnel conducting an examination or a biopsy on a monitor in step S50, along with any information provided by the second instance of artificial intelligence.

Fig. 2 shows a schematic representation of an embodiment of an instance of an artificial intelligence useful for the identification of suspicious areas in images of the larynx. This embodiment of an instance of artificial intelligences may be applicable to the first instance of artificial intelligence as well as to the second instance, and is configured as a convolutional neural network (CNN) having a classifier on the output side. The images are fed into the CNN on the input side and undergo a feature extraction by being processed inside the CNN's convolutional base ("convolution") and pooled ("pooling") according to the known principles of working of convolutional neural networks. The convolution and putting stages may be repeated once or a few times. As a result, of the feature extraction, features of the NBI images that may indicate suspicious areas are identified. These are then set into the classification layer of the CNN, which may be a fully connected layer, or several layers, and processed towards an output layer, in which each node represents a classification that the CNN has been trained for. In the simplest case, there may be only a very few nodes in the output layer that represent the presence or absence of a suspicious area, which may be sufficient for the first instance. In a more sophisticated embodiment applicable for the second instance of artificial intelligence, the output layer may have several nodes representing different diseases, lesion types, severities, etc.

The explanation of the principle using a convolutional neural network is not to be construed as limiting, as other examples of artificial intelligence and machine learning may also be trained and employed for this purpose, such as, e.g., support vector machines, learning vector quantization, or random forest models.

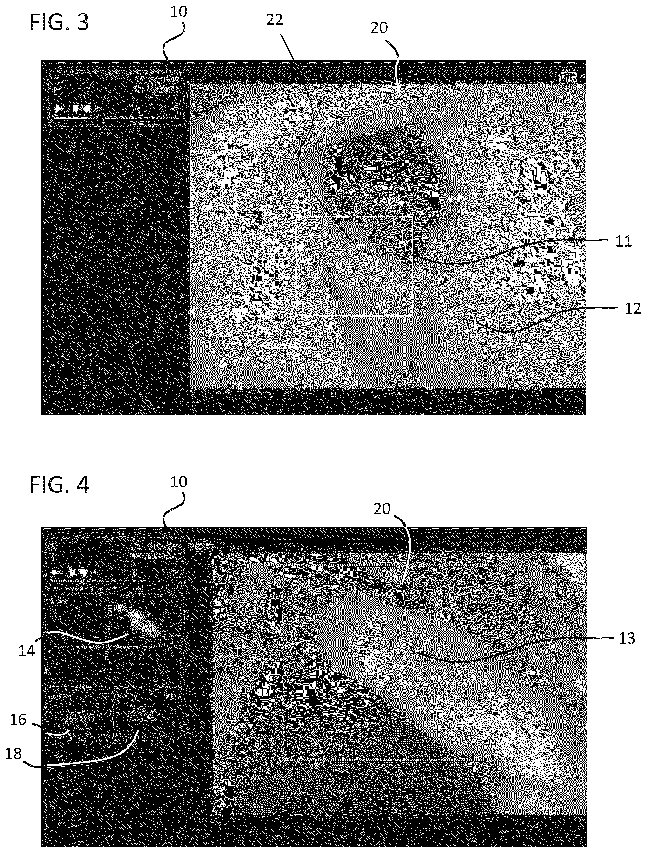

Fig. 3 represents a first embodiment of a GUI 10 representing a first result of the present method. The GUI 10 will be shown on a monitor to a physician performing an endoscopic examination and possibly biopsy of the larynx of a patient. The major part of GUI 10 is used to display an endoscopic image of part of the upper aerodigestive tract including the larynx. An insert in the upper left corner may display patient information as well as the duration of the procedure and an indication of the progress of the examination in the form of a progress bar, above which are displayed symbols signifying anatomical milestones that have been passed during the examination, such as the mouth (diamond shaped symbol), the tonsils (circular symbol) and the larynx (shell shaped symbol). These symbols are highlighted because the endoscope tip has passed or reached these milestones. Other milestones further down have not been reached yet and are still grayed out accordingly.

The endoscopic image of the upper aerodigestive tract is overlaid with rectangular markings of areas that the first instance of artificial intelligence has identified as being suspicious. Each rectangle is accompanied by a percentage number indicating a confidence level of the result. The finding of an abnormality 22 with the highest confidence level of 92% is highlighted as a main marking 11 having a solid border line, whereas the other findings, having lower confidence levels between 52% and 88% are marked with dotted lines as secondary markings 12. Findings with confidence levels below 50% are not displayed. The minimum confidence level for display may be chosen higher or lower than 50%, as the case may be. A lower boundary value will have the benefit of being able to highlight very slight anomalies, at the cost of an increased number of false positive markings. A higher boundary value will reduce the number of false positives at the cost of sensitivity to very slight abnormalities, increasing the risk of missing detecting very early state malignant changes.

Fig. 4 represents a second embodiment of a GUI 10, or a second exemplary configuration of GUI 10. In this configuration, results from the second instance of artificial intelligence are shown overlaid over and next to an endoscopic NBI image of laryngeal tissue 20. Since the NBI image has been processed by the second instance of artificial intelligence trained for this purpose, a suspicious tissue structure 13 has been identified and classified and is surrounded with a rectangular box.

In the left part of GUI 10, several inserts are displayed that provide information about the suspicious area as well as about suggestions that are based on the findings of the instance of artificial intelligence about the suspicious area. The uppermost insert is the same one as shown in Fig. 3, showing general patient and procedure information as well as a progress bar. A second insert below the first insert shows the outline of the suspicious area relative to its position inside the throat in the form of a heat map generated by the first instance of artificial intelligence. As can be seen from the second insert, the suspicious area is located in the upper right part of the throat. Its relative size can also be derived from this graphic representation.

The third insert directly below the heat map display contains two pieces of information, namely a size indicator 16 ("5mm"), and a typization indicator 18 ("SCC", for "Squamous Cell Carcinoma"). These indications are accompanied by confidence level indicators consisting of three bars. In the case of the size indicator 18, two of three bars are highlighted, designating a medium level of confidence for the size evaluation result, whereas the typization indicator has three of three bars highlighted, meaning a high level of confidence in the typization of the abnormality 22.

The marking of a suspicious area that has been identified by the instance of artificial intelligence in NBI images may remain in the display after the imaging has been switched over to white light imaging (WLI), with some lateral movement and magnification is a shift of the location of the endoscope is detected in the images. This is in particular helpful for the actual act of taking biopsy samples from the suspicious area.

Fig. 5 shows exemplary representations of training data for the two instances of artificial intelligence. The uppermost training data are unclassified NBI training images that contain only healthy tissue. Such training data are needed for so-called unsupervised training of the artificial intelligence. The training will prompt the instance of the artificial intelligence to accept any laryngeal tissues and services as on suspicious that conform to these training data. If there is a deviation from the structures that have been trained as being normal, the affected area will show up automatically and be classified as a suspicious area.

The alternative case of so-called supervised learning is based on free classified and be a training images, some of which only display healthy tissue, but others of which contain lesioned or diseased areas, in which such affected areas are indicated and classified, either simply as suspicious or, in higher granularity, regarding their types and severities. The supervised training results in an optimization such that specific nodes in the output layer of a CNN are trained to indicate the various types and severities, respectively.

The third kind of input images indicated in Fig. 5 are new endoscopic NBI images that are captured during examinations and biopsies performed using the pre-trained instance of artificial intelligence and are used to continuously update the training of the artificial intelligence instance, both in unsupervised and in supervised learning. In the case of unsupervised learning, the physician performing an examination may indicate that the present view of the larynx is free from suspicious areas. In that case, the NBI image can be used for further establishing the ground truth of NBI training images for unsupervised learning. Alternatively, in case of the presence of suspicious areas of the physician may confirm the finding and classification of a suspicious area or alter a finding that he has found to be incorrect, so that this image including its correct classification can be used for further supervised training of the instance of artificial intelligence.

Fig. 6 is a schematic representation of a system according to the present disclosure. The system comprises an endoscope 30, which may be a flexible endoscope having an optics and an image sensor at its distal end, or a rigid endoscope with an internal relay optics and an image sensor in its handle or in a separate camera head that is attached to the endoscope. The image sensor is connected to a controller 32 of the system used to control the imaging, lighting and other parameters of an endoscopic examination. The controller 32 may comprise a light source 36 capable of producing white light for WLI and alternative the narrowband lighting for NBI. A light source may also be implemented in the endoscope 30 and controlled by controller 32. The controller 32 furthermore comprises an image analyzer 34 running an instance of artificial intelligence 34 trained to identify and classify suspicious areas in laryngeal tissue according to the previous description. The image analyzer 34 is also configured to provide the NBI images with overlays that visualize or highlight suspicious areas detected by the instance of artificial intelligence. Examples of such overlays are shown and described with respect to Fig. 3, for example.

The overlaid images are displayed using a monitor or display 38, for example in the form of a GUI 10 as shown in Fig. 3, possibly including further information, such as information about the size, type and/or severity of suspicious areas, or suggestions for the number and/or locations of biopsy samples to be taken or about the type of forceps to be used.

Furthermore, the system may include a feedback terminal 40, providing a physician carrying out an examination of the larynx to provide feedback about the findings of the instances of artificial intelligence with respect to suspicious areas, for example confirming or altering such findings. Such feedback may be used for further training of the instances of artificial intelligence, as explained with respect to Fig. 4. Such feedback terminal 14 may be a separate device, such as a computer, or be integrated into the display, as indicated by the dashed line around display 38 and feedback terminal 40, in which case the display has a touchscreen and the GUI 10 displays defined areas of the screen for feedback by touching the touch screen. For example, a touch on an insert area of GUI 10 displaying information about type or severity of a lesion or otherwise suspicious area may open a dialogue for correcting the automatically generated information with the physician's findings. In place of input by touchscreen, the correction may also carried out via voice recognition or other suitable means.

All named characteristics, including those taken from the drawings alone, and individual characteristics, which are disclosed in combination with other characteristics, are considered alone and in combination as important to the invention. Embodiments according to the invention can be fulfilled through individual characteristics or a combination of several characteristics. Features which are combined with the wording "in particular" or "especially" are to be treated as preferred embodiments.

While there has been shown and described what is considered to be embodiments of the invention, it will, of course, be understood that various modifications and changes in form or detail could readily be made without departing from the spirit of the invention. It is therefore intended that the invention be not limited to the exact forms described and illustrated, but should be constructed to cover all modifications that may fall within the scope of the appended claims.

### List of Reference Signs

- 10: GUI
- 11: main marking
- 12: secondary marking
- 13: suspicious tissue structure
- 14: representation of suspicious area
- 16: suggestion for number of biopsy samples
- 18: suggestion for forceps to be used
- 20: laryngeal tissue
- 22: abnormality
- 30: endoscope
- 32: controller
- 34: image analyzer with instance of AI
- 36: light source
- 38: display
- 40: feedback terminal

## Claims

1. Endoscopic medical imaging analysis and manipulation method, the method comprising:
Capturing endoscopic images of laryngeal, pharyngeal and/or surrounding tissue structures during examination of the larynx, pharynx and/or surrounding tissue using an endoscope inserted through a patient's nose or mouth,
Feeding the captured endoscopic images to two instances of artificial intelligences, a first of which having been trained to identify suspicious areas of laryngeal, pharyngeal and/or surrounding tissue structures showing signs of alterations from healthy laryngeal, pharyngeal and/or surrounding tissue and a second of which having been trained to detect and classify abnormalities in the mucosal layer or the tissue of the larynx, pharynx and/or surrounding tissue,
Overlaying the captured endoscopic images with one or more markings indicating an area or areas indicated by the first instance of artificial intelligence as suspicious, and
Displaying the overlaid captured endoscopic images on a monitor, together with information provided by the second instance of artificial intelligence.

2. The endoscopic medical imaging analysis and manipulation method of claim 1, wherein the endoscopic images are one of WLI images, NBI images and a mix of WLI images and NBI images.

3. The endoscopic medical imaging analysis and manipulation method of claim 1 or 2,
wherein the first instance of an artificial intelligence being a convolutional neural network (CNN) having a classifier, the CNN having been trained by at least one of supervised and unsupervised learning of a multitude of endoscopic images of laryngeal, pharyngeal and/or surrounding tissue structures s to classify suspicious areas of laryngeal, pharyngeal and/or surrounding tissue in the captured endoscopic images and/or
wherein the second instance of an artificial intelligence being a convolutional neural network (CNN) having a classifier, the CNN having been trained by supervised learning of a multitude of pre-classified endoscopic images of laryngeal, pharyngeal and/or surrounding tissue structures to classify suspicious areas of laryngeal, pharyngeal and/or surrounding tissue in the captured endoscopic images according to type and/or severity of abnormalities in the suspicious areas, wherein in particular the type of abnormalities include one or more of cancerous and benign lesions, cancerous and benign blood vessel morphologies, cancerous and benign blood vessel densities, cancerous and benign vascular patterns, and cancerous and benign structure of the mucosal surface.

4. The endoscopic medical imaging analysis and manipulation method of one of claims 1 to 3, wherein the overlays being created as rectangles or outlines, and/or wherein the overlays being adjustable based on the movements of the user.

5. The endoscopic medical imaging analysis and manipulation method of one of claims 1 to 4, wherein a live measure of classification accuracy by the first instance of artificial intelligence and/or the second instance of artificial intelligence being displayed, in particular based on factors such as the distance of the endoscope from the lesion, the angulation of the lengths, and clarity of the image.

6. The endoscopic medical imaging analysis and manipulation method of one of claims 1 to 5, wherein the first instance of artificial intelligence or the second instance of artificial intelligence being trained to identify image defects that necessitate an adjustment or cleaning of the endoscope lens or cleaning of the anatomical area, and a user is notified of a suggestion to adjust or clean the endoscope lens or to clean the anatomical area in the case of the occurrence of such image defects.

7. The endoscopic medical imaging analysis and manipulation method of one of claims 1 to 6, wherein any one or both of the instances of artificial intelligence being designed to continuously learn from new images, wherein in particular the new images being classified while being produced by a physician performing the examination through confirming, altering or adding findings with respect to suspicious areas or classifications.

8. The endoscopic medical imaging analysis and manipulation method of one of claims 1 to 7, wherein the second instance of artificial intelligence being trained to estimate the size of a lesion and to provide an estimation of its length, the size information in particular being used to produce a two-dimensional lesion map of the anatomical area.

9. Endoscopic medical imaging analysis und manipulation system comprising a video endoscope suited to be fed through a patient's mouth or nose for laryngeal and/or pharyngeal examination, an image analyzer connected to the video endoscope for receiving endoscopic images from the endoscope, the image analyzer having a first instance of an artificial intelligence trained to identify suspicious areas of laryngeal, pharyngeal and/or surrounding tissue structures showing signs of alterations from healthy laryngeal, pharyngeal and/or surrounding tissue and a second instance of artificial intelligence having been trained to detect and classify abnormalities in the mucosal layer or the tissue of the larynx, pharynx or surrounding tissue, the image analyzer further being configured to overlay, the captured endoscopic images with a marking indicating areas indicated by the instance of artificial intelligence as suspicious, and a monitor connected to the image analyzer for displaying endoscopic images provided by the image analyzer, together with information provided by the second instance of artificial intelligence.

10. The endoscopic medical imaging analysis and manipulation system of claim 9,
wherein the first instance of an artificial intelligence being a convolutional neural network (CNN) having a classifier, the CNN having been trained by at least one of supervised and unsupervised learning of a multitude of endoscopic images of laryngeal, pharyngeal and/or surrounding tissue structures to classify suspicious areas of laryngeal, pharyngeal and/or surrounding tissue in the captured endoscopic images, and/or
wherein the second instance of an artificial intelligence being a convolutional neural network (CNN) having a classifier, the CNN having been trained by supervised learning of a multitude of pre-classified endoscopic images of laryngeal, pharyngeal and/or surrounding tissue structures to classify suspicious areas of laryngeal, pharyngeal and/or surrounding tissue in the captured endoscopic images according to type and/or severity of abnormalities in the suspicious areas, wherein in particular the type of abnormalities include one or more of cancerous and benign lesions, cancerous and benign blood vessel morphologies, cancerous and benign blood vessel densities, cancerous and benign vascular patterns, and cancerous and benign structure of the mucosal surface.

11. The endoscopic medical imaging analysis and manipulation system of claim 9 or 10, wherein the overlays being adjustable based on the movements of the user.

12. The endoscopic medical imaging analysis and manipulation system of one of claims 9 to 11, wherein a live measure of classification accuracy by the first instance of artificial intelligence and/or the second instance of artificial intelligence being displayed, in particular based on factors such as the distance of the endoscope from the lesion, the angulation of the lengths, and clarity of the image.

13. The endoscopic medical imaging analysis and manipulation system of claims 9 to 12, wherein the first instance of artificial intelligence or the second instance of artificial intelligence being trained to identify image defects that necessitate an adjustment or cleaning of the endoscope lens or cleaning of the anatomical area, and a user is notified of a suggestion to adjust or clean the endoscope lens or to clean the anatomical area in the case of the occurrence of such image defects, and/or wherein the second instance of artificial intelligence being trained to estimate the size of a lesion and to provide an estimation of its length, the size information in particular being used to produce a two-dimensional lesion map of the anatomical area.

14. The endoscopic medical imaging analysis and manipulation system of claims 9 to 13, wherein any one or both of the instances of artificial intelligence being designed to continuously learn from new images, wherein in particular the new images being classified while being produced by a physician performing the examination through confirming, altering or adding findings with respect to suspicious areas or classifications.

15. Computer program stored on a non-volatile medium, the computer program being designed to perform the steps of any of claims 1 to 8, in particular when run on an image analyzer of a system according to any of claims 9 to 14.
